# EUROPEAN PATENT APPLICATION

(11) **EP 4 656 195 A1**
(43) Date of publication of application: **03.12.2025**
(21) Application number: 23940228.2
(22) Date of filing: 26.06.2023
(51) Int. Cl.: A61K 31/7105, A61P 1/16, C12N 5/071

(54) **USE OF NUCLEIC ACID MOLECULE IN PREPARATION OF DRUG FOR TREATING LIVER DISEASE**

(30) Priority: 05.06.2023 CN 202310654911
(71) Applicant: GUANGXIU-GAOXIN LIFE SCIENCES CO., LTD. HUNAN, Changsha, Hunan 410205 (CN)
(72) Inventor: LIN, Ge, Changsha, Hunan 410205 (CN); SUN, Yi, Changsha, Hunan 410205 (CN); LU, Guangxiu, Changsha, Hunan 410205 (CN); LI, Jie, Changsha, Hunan 410205 (CN)
(74) Representative: Bryn Aarflot AS
(86) International application number: PCT/CN2023/102374
(87) International publication number: WO 2024/250347

(57) **Abstract**

The present disclosure relates to the use of a nucleic acid molecule in preparation of a drug for treating a liver disease. The nucleic acid molecule satisfies one or more of the following conditions: comprising any one or more of miR-302s; comprising mimics or derivatives thereof; comprising a miRNA precursor which can be processed into any one or more of miR-302s and mimics or derivatives thereof; comprising a polynucleotide fragment which can be transcribed in a host to form a miRNA precursor; and comprising an expression vector.

## Description

### RELATED APPLICATIONS

This application claims priority to Chinese patent application No. 2023106549115, filed on June 5, 2023, entitled "USE OF NUCLEIC ACID MOLECULE IN PREPARATION OF DRUG FOR TREATING LIVER DISEASE", which is hereby incorporated by reference in its entirety.

### TECHNICAL FIELD

The present disclosure relates to the fields of biology and medicine, in particular to use of a nucleic acid molecule in preparation of a medicament for treating a liver disease.

### BACKGROUND

MicroRNA (miRNA) is a type of endogenous single-stranded small RNA with a length of about 20-24 bases, and generated through the processing of hairpin-structured single-stranded RNA precursors (pre-miRNAs) of about 70-90 bases by the Dicer enzyme. The seed sequence of miRNA, defined as nucleotides 2 to 8 at the 5' end of miRNA, is the most evolutionarily conserved fragment on miRNA. With the seed sequence, miRNA completely or incompletely base-pairs with the 3' non-coding region of the target gene mRNA, causing the target gene mRNA to degrade or inhibiting its protein translation, thereby participating in gene regulation. Each miRNA can regulate the expression of multiple target genes, and multiple miRNAs can also regulate the expression of a single gene. It is estimated that miRNA regulates one-third of human genes and has various important functions within cells. Studies have found that miRNA plays significant roles in regulating biological processes such as organism development, cell proliferation, differentiation, and death, etc., and is involved in the occurrence and development of various diseases such as tumors, cardiovascular, neurological, and immune diseases, etc. It is reported that miRNA can serve as a diagnostic marker for certain diseases. For example, miR-122 is highly expressed in the serum of patients with acute liver failure, even abnormally elevates before the conventional liver function indicators ALT and AST, and can serves as an early diagnostic molecule. In recent years, research has begun to explore miRNA as a novel approach for disease treatment.

In view of this, the present disclosure is hereby proposed.

### SUMMARY

On this basis, according to various embodiments of the present disclosure, technical schemes of use of a nucleic acid molecule in preparation of a medicament for treating a liver disease are provided as follows.

In a first aspect of the present disclosure, the present disclosure provides use of a nucleic acid molecule in preparation of a medicament for treating a liver disease.

The nucleic acid molecule satisfies one or more of the conditions as shown in items (a) to (e):
(a) the nucleic acid molecule includes any one or more of miR-302 members;
(b) the nucleic acid molecule comprises a mimic or derivative of any one or more of miR-302 members, wherein the mimic or derivative has a core fragment as set forth in SEQ ID No. 1 and has the same or similar function as any one or more of miR-302 members;
(c) the nucleic acid molecule includes a miRNA precursor, the miRNA precursor being processable into any one or more of miR-302 members, the mimic or derivative thereof in a host;
(d) the nucleic acid molecule includes a polynucleotide fragment, the polynucleotide fragment being transcribable in the host to form the miRNA precursor; and
(e) the nucleic acid molecule includes an expression vector, and the expression vector contains the polynucleotide fragment.

In some embodiments of the present disclosure, the nucleic acid molecule is one or more selected from the group consisting of miR-302a-3p, miR-302b-3p, miR-302c-3p and miR-302d-3p.

In some embodiments of the present disclosure, the liver disease is acute liver failure.

In some embodiments of the present disclosure, the medicament includes the nucleic acid molecule and a pharmaceutically acceptable excipient.

In some embodiments of the present disclosure, the medicament is in the form of a tablet.

In some embodiments of the present disclosure, the medicament is in the form of a capsule.

In some embodiments of the present disclosure, the medicament is in the form of granules.

In some embodiments of the present disclosure, the medicament is in the form of a suspension.

In some embodiments of the present disclosure, the medicament is in the form of an oral solution.

In some embodiments of the present disclosure, the medicament is in the form of an injection.

In a second aspect of the present disclosure, an in vitro method for protecting hepatocytes is provided. The method includes culturing hepatocytes in a culture medium supplemented with the nucleic acid molecule defined in the first aspect.

In some embodiments of the present disclosure, the hepatocytes are hepatocytes damaged by D-galactosamine (D-gal) induction.

In some embodiments of the present disclosure, the hepatocytes are mouse primary hepatocytes (mPH).

In some embodiments of the present disclosure, the nucleic acid molecule has a concentration of 10 nM to 100 nM.

Details of one or more embodiments of the present disclosure are set forth in the following description. Other features, objects, and advantages of the present disclosure will become apparent from the description and claims of the present disclosure.

### BRIEF DESCRIPTION OF THE DRAWINGS

The below will briefly describe drawings to be used in the description of embodiments of the present disclosure to more clearly illustrate the technical solutions in embodiments of the present disclosure. Obviously, the drawings in the following description are only some embodiments of the present disclosure and other drawings can also be obtained by those skilled in the art based on these drawings without creative work.
FIG. 1 shows the cell viability test results showing that the miR-302s mimics of an embodiment of the present disclosure have the ability to protect liver cells from damage *in vitro.*
FIG. 2 shows the results of dual luciferase reporter gene experiments for miR-302b-3p of an embodiment of the present disclosure targeting Bcl2l11;
FIG. 3 shows the results of Western blot detection and cell viability tests in the in vitro validation experiments for miR-302b-3p of an embodiment of the present disclosure to protect hepatocytes by targeting Bcl2l11.
FIG. 4 is a schematic diagram of staining of mitochondrial membrane potential (MMP), reactive oxygen species (ROS) and apoptotic protein Caspase-3 cleavage form cl-Caspase3 (cl-Casp3) of mPH in different treatment groups in the in vitro validation experiments for miR-302b-3p of an embodiment of the present disclosure to protect hepatocytes by targeting Bcl2l11.
FIG. 5 is a survival curve graph of the modeling control group (Control) and the miR-302b-3p treatment group in the in vivo experiments in which acute liver failure mice is protected by miR-302b-3p of an embodiment of the present disclosure.
FIG. 6 shows the serum ALT and AST test results of the modeling control group (Control) and the miR-302b-3p treatment group in the in vivo experiments in which acute liver failure mice is protected by miR-302b-3p of an embodiment of the present disclosure.
FIG. 7 is a histopathological diagram of the modeling control group (Control) and the miR-302b-3p treatment group in the in vivo experiments in which acute liver failure mice is treated with miR-302b-3p of an embodiment of the present disclosure.
FIG. 8 shows the results of immunoblotting of liver tissue proteins of the modeling control group (Control) and the miR-302b-3p treatment group in the in vivo experiments in which acute liver failure mice is treated with miR-302b-3p of an embodiment of the present disclosure.

### DETAILED DESCRIPTION

The technical solutions of the embodiments of the present disclosure is hereafter clearly and completely described with reference to the accompanying drawings in the embodiments of the present disclosure. The described embodiments are merely some rather than all of the embodiments of the present disclosure. All other embodiments obtained by a person of ordinary skill in the art based on the embodiments of the present disclosure without creative efforts shall fall within the protection scope of the present disclosure.

Unless otherwise defined, all technical and scientific terms used herein have the same meaning as commonly understood by those skilled in the art to which the present disclosure belongs. The terms used herein in the specification of the present disclosure are for the purpose of describing specific embodiments only and are not intended to limit the present disclosure.

### Terminology

Unless otherwise indicated or contradicted, terms or phrases used herein have the following meanings:
As used herein, the scope of the terms "and/or" and "or/and" as used herein includes any item in two or more associated listed items, and also includes any and all combinations of the associated listed items. The any and all combinations include a combination of any two associated listed items, any more associated listed items, or all associated listed items. It should be noted that when at least two conjunction pairings selected from "and/or" and "or/and" are used to connect at least three items, the technical solution of the present disclosure undoubtedly includes the solutions connected by "logical AND" and the solution connected by "logical OR". For example, the term "A and/or B" includes three parallel schemes: A, B, and "a combination of A and B". For another example, a technical solution of "A and/or B and/or C and/or D" includes any item in A, B, C, and D (i.e., a technical solution connected by "logical OR"), and also includes any and all combinations of A, B, C, and D, which means including a combination of any two items or any three items in A, B, C, and D, and also including a four-item combination of A, B, C, and D (i.e., a technical solution connected by "logical AND").

Unless otherwise specified, the terms "a plurality of", "multiple" "multiple times", "multi-" and the like related in the present disclosure mean greater than 2 or equal to 2 in number. For example, "one or more" means one or two or more.

As used herein, the terms "a combination thereof", "any combination thereof", and the like include all suitable combinations of any two or more of the listed items.

Herein, the word "suitable" in "suitable combination", "suitable mode", "any suitable mode", and the like, is construed as long as it enables the implementation of the technical solution of the present disclosure, resolves the technical problem of the present disclosure, and achieves the anticipated technical effect of the present disclosure.

Herein, the terms "preferably", "better", "preferable", and "preferred" are only used to describe embodiments or examples with a better effect. It should be understood that these terms do not limit the protection scope of the present disclosure.

Herein, the terms "further", "still further", "specifically", and the like are used for descriptive purposes to indicate differences in contents, but should not be construed as limiting the protection scope of the present disclosure.

Herein, the terms "optionally" and "optional" refer to something that is not necessary, i.e., any one selected from the two parallel schemes in which the listed items is "present" or "absent". If multiple "optionally" appear in a technical solution, unless otherwise specified and in the absence of contradictions or interdependencies, each of the "optionally" is independent from each other.

In the present disclosure, the terms "first", "second", "third", "fourth", etc. in "first aspect", "second aspect", "third aspect", "fourth aspect", etc. are used for descriptive purposes only and are not to be construed as indicating or implying a relative importance or quantity, nor as implying any indication of the importance or quantity of the technical features indicated. Moreover, the "first", "second", "third", "fourth", etc. are used merely for purposes of non-exhaustive enumeration and description, and should not be construed as a limitation of quantity in closed-ended manner.

In the present disclosure, the technical features described in open-ended manner include a closed-ended technical solution consisting of the listed features, and also include an open-ended technical solution including the listed features.

In the present disclosure, when referring to a numerical interval (i.e., a numerical range), unless otherwise specified, the distribution of optional values within the numerical interval is considered to be continuous and includes both the endpoints of the numerical interval (i.e., the minimum and maximum values) and each value between these two endpoints. Unless otherwise specified, when the numerical interval only refers to the integers within the numerical interval, it includes the two endpoint integers of the numerical range, and each integer between the two endpoints. Herein, it is equivalent to directly listing each integer. For example, t is an integer selected from 1 to 10, indicating that t is any integer selected from the integer group consisting of 1, 2, 3, 4, 5, 6, 7, 8, 9 and 10. In addition, when multiple ranges are provided to describe features or characteristics, these ranges can be combined. In other words, unless otherwise specified, the ranges disclosed herein should be understood to include any and all sub-ranges included therein.

Unless otherwise specified, the temperature parameter in the present disclosure is allowed to be constant temperature treatment or to vary within a certain temperature interval. It should be understood that the constant temperature treatment allows the temperature to fluctuate within the accuracy range controlled by the instrument. It is allowed to fluctuate in a range such as ±5° C., ±4° C., ±3° C., ±2° C., and ±1° C.

In the present disclosure, %(w/w) and wt% both represent weight percentage, %(v/v) refers to volume percentage, and %(w/v) refers to mass volume percentage.

All documents mentioned in the present disclosure are incorporated by reference, just as each document is cited separately as a reference. The entire content and purpose of the cited references related in the present disclosure are incorporated by reference unless they conflict with the objectives and/or technical solutions of the present disclosure. When a cited reference is related in the present disclosure, definitions of relevant technical features, terms, nouns, phrases, and the like in the cited reference are also incorporated herein. When a cited reference is related in the present disclosure, the examples, and preferred embodiments of the cited relevant technical features are also incorporated in the present disclosure as references, but only to the extent that they enable the implementation of the present disclosure. It should be understood that when a reference conflicts with the description of the present disclosure, the present disclosure shall prevail or the reference shall be adaptively modified according to the description of the present disclosure.

MiR-302 family includes members such as miR-302a-3p, miR-302b-3p, miR-302c-3p, miR-302d-3p and miR-367-3p, which are highly expressed miRNAs in embryonic stem cells. Among them, four members, miR-302a-3p, miR-302b-3p, miR-302c-3p and miR-302d-3p, have the same seed sequence and are generally collectively referred to as miR-302s, suggesting that they have the same or similar functions. The miR-302 family has multiple functions such as regulating reprogramming, inhibiting tumorigenesis, proliferation, invasion and metastasis, maintaining the self-renewal and pluripotency of stem cells, promoting regeneration and repair, as well as inhibiting inflammation and regulating anti-infection immune responses. The miR-302 family has different functions for different cell types. For example, miR-302b/c can promote the proliferation of local epithelial progenitor cells to regenerate alveolar epithelial cells, thereby improving lung function, accelerating host recovery and improving survival rate, and thus exhibiting a therapeutic effect on mice infected with Streptococcus pneumoniae. In mesenchymal stem cells, the miR-302s promotes the proliferation of human adipose tissue-derived mesenchymal stem cells by inhibiting the expression of cyclin-dependent kinase inhibitor 1A (CDKN1A/p21) and inflammatory chemokine CCL5, and protects cells from oxidant-induced apoptosis. The miR-302 family inhibits the Hippo pathway by inhibiting kinases Mst1, Lats2 and Mob1b, thereby inducing cardiomyocyte proliferation and promoting the regeneration and repair of damaged myocardium. However, the role of the miR-302s in hepatocytes is still unclear, and there are no reports on its related functions in liver diseases. The present disclosure provides an application of the miR-302s and related nucleic acid molecules in inhibiting hepatocyte apoptosis signaling pathways and treating acute liver failure, providing a new idea for the treatment of clinical liver diseases.

### A first aspect of the present disclosure

The present disclosure relates to use of a nucleic acid molecule in preparation of a medicament for treating a liver disease;
the nucleic acid molecule satisfies one or more of the conditions as shown in items (a) to (e):
   (a) the nucleic acid molecule includes any one or more of miR-302 members;
   (b) the nucleic acid molecule comprises a mimic or derivative of any one or more of miR-302 members, wherein the mimic or derivative has a core fragment as set forth in SEQ ID No. 1 and has the same or similar function as any one or more of miR-302 members;
   (c) the nucleic acid molecule includes a miRNA precursor, the miRNA precursor being processable into any one or more of miR-302 members, mimics or derivatives thereof in a host;
   (d) the nucleic acid molecule includes a polynucleotide fragment, the polynucleotide fragment being transcribable in the host to form the miRNA precursor;
   (e) the nucleic acid molecule includes an expression vector, and the expression vector contains the polynucleotide fragment.
SEQ ID No.1:5' -AAGUGCU-3' _{∘}

In an example, the nucleic acid molecule is one or more selected from the group consisting of miR-302a-3p, miR-302b-3p, miR-302c-3p and miR-302d-3p.

In the present disclosure, "medicament" includes any agent, compound, composition or mixture that provides a physiological and/or pharmacological effect in vivo or in vitro, and often provides a beneficial effect. The range of physiological and/or pharmacological effects produced by the "medicament" in vivo is not particularly limited, and may be systemically effective or locally effective. The activity of the "medicament" is not particularly limited, and the medicament may be an active substance that can interact with other substances or an inert substance that does not interact with other substances.

In an example, the medicament may be a liquid formulation or a solid formulation. Liquid formulation refers to a formulation containing a liquid phase, such as, by way of non-limiting examples, a solution, a suspension, an emulsion, or the like. Non-limiting examples of solid formulations are a tablet, a capsule, a granule, a pill, or the like.

In an example, the medicament may be an oral formulation, an injection, a drop, a patch, a tube feed formulation, etc., depending on the mode of administration.

In an example, the excipients selected may be different depending on the dosage form.

In the present disclosure, the excipients include, but are not limited to, mannitol, sorbitol, sodium metabisulfite, sodium bisulfite, sodium thiosulfate, cysteine hydrochloride, thioglycolic acid, methionine, vitamin C, EDTA disodium salt, sodium calcium EDTA, monovalent alkali metal carbonates, acetate salts, phosphate salts or aqueous solutions thereof, hydrochloric acid, acetic acid, sulfuric acid, phosphoric acid, amino acids, sodium chloride, potassium chloride, sodium lactate, xylitol, maltose, glucose, fructose, dextran, glycine, starch, sucrose, lactose, mannitol, silicon derivatives, cellulose and derivatives thereof, alginates, gelatin, polyvinylpyrrolidone, glycerol, Tween 80, agar, calcium carbonate, calcium bicarbonate, surfactants, polyethylene glycol, cyclodextrin, phospholipid materials, kaolin, tale, calcium stearate, and magnesium stearate.

In the present disclosure, the term "pharmaceutically acceptable" refers to any one or any suitable combination of those ligands, materials, compositions, and/or dosage forms that are suitable, within the scope of sound medical judgment, for administration to a patient and are commensurate with a reasonable benefit/risk ratio.

In the present disclosure, the term "pharmaceutically acceptable excipient" refer to pharmaceutically acceptable materials, compositions, or vehicles, such as liquid or solid fillers, diluents, excipients, solvents, or encapsulating materials. As used herein, the wording "pharmaceutically acceptable carrier" include buffers, sterile water for injection, solvents, dispersion media, coatings, antibacterial and antifungal agents, isotonic agents, absorption delaying agents, and the like, that are compatible with pharmaceutical administration. Each carrier must be "pharmaceutically acceptable" in the sense of being compatible with the other ingredients of the formulation and harmless to the patient. Suitable examples include, but are not limited to: (1) sugars, such as lactose, glucose, and sucrose; (2) starches, such as corn starch, potato starch, and substituted or unsubstituted P-cyclodextrin; (3) cellulose and derivatives thereof, such as sodium carboxymethylcellulose, ethylcellulose, and cellulose acetate; (4) powdered tragacanth; (5) malt; (6) gelatin; (7) talc; (8) excipients, such as cocoa butter and suppository waxes; (9) oils, such as peanut oil, cottonseed oil, safflower oil, sesame oil, olive oil, corn oil, and soybean oil; (10) diols, such as propylene glycol; (11) polyols, such as glycerol, sorbitol, mannitol, and polyethylene glycol; (12) esters, such as ethyl oleate and ethyl laurate; (13) agar; (14) buffers, such as magnesium hydroxide and aluminum hydroxide; (15) alginic acid; (16) pyrogen-free water; (17) isotonic saline; (18) Ringer's solution; (19) ethanol; (20) phosphate buffered saline; and (21) other non-toxic compatible substances used in pharmaceutical formulations.

In an example, the medicament includes an effective amount of the nucleic acid molecule.

In the present disclosure, the term "effective amount" refers to the dose of the component corresponding to this term that achieves the treatment, prevention, alleviation and/or relief of a specific disease, disorder, and/or symptom in a subject. In the present disclosure, unless otherwise specified, this term refers to the dose that achieves the treatment, prevention, alleviation and/or relief of a liver disease, disorder, and/or symptom.

In the present disclosure, "subject" is an animal, preferably a mammal, and further preferably a human. A subject includes, but is not limited to, consumers of healthcare products and patients with a disease, a disorder and/or a symptom. The subject of the present disclosure is preferably a mammal. The term "mammal" refers primarily to warm-blooded vertebrate mammals, including but not limited to, for example, cats, dogs, rabbits, bears, foxes, wolves, monkeys, deers, murine (e.g., rats and mice), pigs, cattle, sheep, horses, humans, etc, preferably primates, more preferably humans.

In an example, the subject is a mammal.

In an example, the subject is a human or a mouse.

In the present disclosure, the "patient" refers to an animal, preferably a mammal, more preferably a human. The term "mammal" refers primarily to warm-blooded vertebrate mammals, including but not limited to, for example, cats, dogs, rabbits, bears, foxes, wolves, monkeys, deers, murine, pigs, cattle, sheep, horses, humans, etc, preferably primates, more preferably humans.

### A second aspect of the present disclosure

The present disclosure provides an in vitro method for protecting hepatocytes, the method including culturing hepatocytes using a culture medium supplemented with the nucleic acid molecule defined in the first aspect.

In an example, the hepatocytes are hepatocytes damaged by D-gal induction.

In an example, the hepatocytes are mPH. In some embodiments, the nucleic acid molecule has a concentration of 10 nM to 100 nM, for example, 10, 20, 30, 40, 50, 60, 70, 80, 90, 100 nM.

The embodiments of the present disclosure will be described in detail below with reference to the examples. It should be understood that these examples are only for illustrative purposes and not intended to limit the scope of the present disclosure. The experimental procedures without specific conditions noted in the following examples are preferably conducted according to the guidelines given in the present disclosure, and may be performed according to the experimental manual or routine conditions in the art, according to the conditions suggested by the manufacturer, or according to the experimental procedures known in the art.

In the examples described below, the measurement parameters related to the components of the raw materials may be slightly deviated from the weighing accuracy range, unless otherwise specified. When temperature and time parameters are related to, acceptable deviation caused by the instrument test accuracy or operational accuracy is allowed.

### I. In Vitro Functional Identification of miR-302s

### 1. miR-302s mimics

miR-302 mimics, which are mimics for cell miR-302s, and their control mimic NCwere purchased from Guangzhou RiboBio Co., Ltd.
mimic NC (SEQ ID No.2): UUUGUACUACACAAAAGUACUG
miR-302a-3p (SEQ ID No.3): UAAGUGCUUCCAUGUUUUGGUGA
miR-302b-3p (SEQ ID No.4): UAAGUGCUUCCAUGUUUUAGUAG
miR-302c-3p (SEQ ID No.5): UAAGUGCUUCCAUGUUUCAGUGG
miR-302d-3p (SEQ ID No.6): UAAGUGCUUCCAUGUUUGAGUGU

### 2. Protective Effects of miR-302s on Hepatocytes In Vitro

mPHs obtained by in situ perfusion were cultured in William's Medium E medium containing 10% (v/v) fetal bovine serum, 1% (v/v) double antibiotics (penicillin and streptomycin), and 1% (w/v) glutamine. An in vitro cell model simulating in vivo liver injury was constructed by stimulating the mPHs with D-gal. For nucleic acid molecule transfection, the medium did not contain double antibiotics, and 50 nM of each of the aforementioned four miR-302 mimics or 100 nM of their control mimic NC were added. After culturing for 24 hours, cell viability was determined using a lactate dehydrogenase cytotoxicity detection kit to determine the in vitro effectiveness of the miRNA mimics. The results were expressed using the following formula: Cell viability (%) = (Absorbance of treated sample - Absorbance of sample control well) / (Absorbance of enzyme activity of untreated cells - Absorbance of sample control well) × 100.

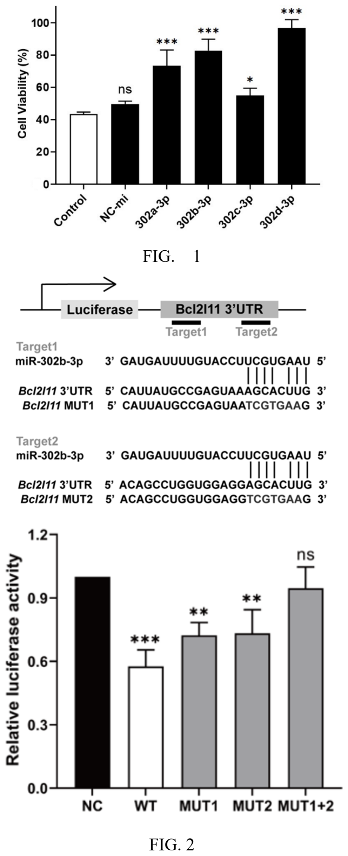

The results showed that all four miR-302s had a protective effect for mPH and could improve the cell viability of mPH, among which miR-302a-3p, miR-302b-3p , and miR-302d-3p was particularly effective. The results are shown in Figure 1.

### II. Targeting Bcl2l11 with miR-302s to Regulate the Hepatocyte Apoptotic Signaling Pathway

### 1. Identification of miR-302s Target Genes

Because the seed sequences of miR-302s are completely identical, the targeting relationship between miR-302s and Bcl2l11 was predicted using the ENCORI, Targetscan, and miRDB websites. The prediction results from all three websites indicated that Bcl2l11 is a target gene of miR-302s in mice. One of the members, miR-302b-3p, was selected for a dual-luciferase reporter gene assay. For the two sites of miR-302b-3p binding to Bcl2l11, two single-site mutant (MUT1 and MUT2) and one double-site mutant (MUT1+2) plasmid vectors were constructed as well as the wild-type plasmid (WT) as a control. The miR-302b-3p mimic was co-transfected with each of the above plasmids into 293T cells. After culturing for 48 hours, the luminescence value of luciferase was determined.

The results are shown in FIG. 2. miR-302b-3p significantly inhibited the luciferase activity of the wild-type plasmid. The inhibitory effect on the luciferase activity of the two single-mutant vectors was comparable and slightly lower than that of the wild-type plasmid. However, the luciferase activity of the double-mutant plasmid was not affected. The results indicate that Bcl2l11 is a target gene of miR-302b-3p.

### 2. miR-302b-3p Primarily Regulating the Hepatocyte Apoptotic Signaling Pathway by Targeting Bcl2l11

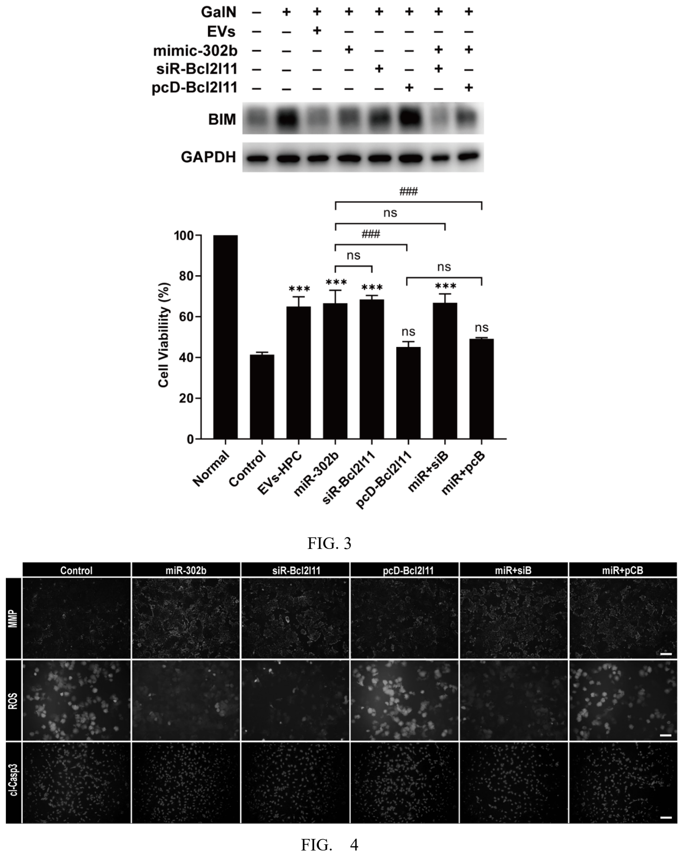

Small interfering RNA (siRNA) against Bcl2l11 (denoted as siR-Bcl2l11 or siB in the figure) and an overexpression plasmid (the Bcl2l11 coding sequence inserted into the pcDNA3.1 vector for target gene overexpression, denoted as pcD-Bcl2l11 or pcB in the figure) were used to knock down and overexpress the target gene Bcl2l11, respectively. Using an in vitro model of D-gal-induced hepatocyte injury, cell viability was determined under different treatment conditions (single treatment groups: EVs-HPC, miR-302b-3p, siR-Bcl2l11, pcD-Bcl2l11; combination groups: miR+siB, miR+pcB). The results are shown in FIG. 3.

EVs-HPC refers to exosomes secreted by embryonic stem cell-induced hepatic progenitor cells.

Cells from the above treatment groups were tested using a cleaved caspase-3 (cl-Caspase3) antibody, a mitochondrial membrane potential (MMP) detection kit, and a reactive oxygen species (ROS) detection kit. The results are shown in FIG. 4.

With reference to FIG. 3 and FIG. 4, the results showed that after D-gal-induced injury, the expression of the Bcl2l11 protein (BIM) in mPH significantly increased, the MMP of hepatocytes decreased, large amounts of ROS were produced, and the apoptotic effector protein Caspase-3 was cleaved and activated, leading to hepatocyte apoptosis and eventual death. miR-302b-3p could significantly inhibit the expression of BIM, maintain the MMP of hepatocytes, reduce the production of ROS, and inhibit the cleavage and activation of Caspase-3, thereby suppressing hepatocyte death. The protective effect of miR-302b-3p on hepatocytes was similar to that observed after knocking down Bcl2l11 using siRNA. The miR-302b-3p in combination with siR-Bcl2l11 did not show an additive effect. However, overexpression of Bcl2l11 using pcD-Bcl2l11 significantly weakened the protective effect of miR-302b-3p on hepatocytes. The above results indicate that miR-302b-3p primarily exerts its protective effect on hepatocytes by inhibiting the mitochondrial apoptotic signaling pathway through targeting the protein expression level of Bcl2l11.

### III. Acute Liver Failure Mouse Modeling and Treatment

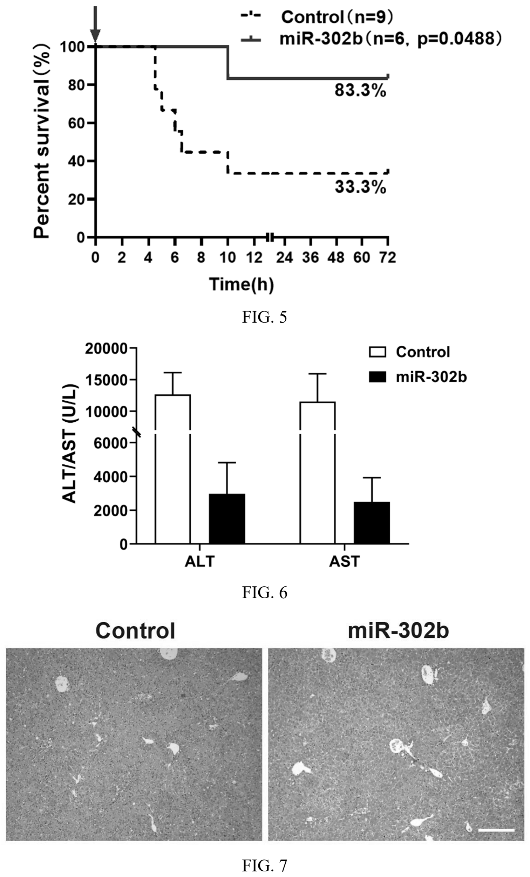

1. Experimental Animals: SPF male Kunming mice weighing 18-22 g were purchased from Hunan Slac Jingda Experimental Animal Co., Ltd. (Production License No.: SYXK (Xiang) 2020-0006). The mice were fed a standard mouse diet with ad libitum access to filtered tap water. Ambient conditions were maintained at a temperature of 22°C ± 1°C and a humidity of 65% ± 5% under a 12-hour light/dark cycle. The animal experimental protocol was reviewed and approved by the Animal Ethics Committee of Central South University. Experimental procedures were strictly conducted in compliance with the National Standard of the People's Republic of China: Guidelines for the Welfare and Ethical Review of Laboratory Animals (GB/T 35892-2018).
2. miR-302b-3p mimics: miR-302b-3p agomir, which is a mimic for miR-302s, for animal experiments were purchased from Guangzhou RiboBio Co., Ltd. Physiological saline served as the control for miR-302b-3p agomir. miRNA agomir is a specially chemically modified miRNA agonist. The sequence of miR-302b-3p agomir is identical to that of miR-302b-3p mimic.
3. Modeling and Treatment: An acute liver failure mouse model was established by intraperitoneal injection of D-galactosamine (D-gal) in combination with lipopolysaccharide (LPS). Treatment was performed via tail vein injection of 10 nmol miR-302b-3p agomir. Survival was monitored for 72 hours, and survival curves were plotted. Results are shown in FIG. 5. The results showed that compared with the untreated control group, miR-302b-3p agomir exerted a protective effect in acute liver failure mice, significantly improving their survival rates and considerably slowing their death rate of.

### IV. Treatment Analysis

### (1) Serum ALT/AST Levels and Histological Observation

ALT and AST levels were measured using a standard clinical autoanalyzer at Hunan Guangxiu Hospital. As shown in FIG. 6, compared with the control group, the miR-302b-3p agomir treatment group exhibited significantly reduced serum ALT and AST levels, indicating markedly improved liver function.

### (2) Histopathological Analysis:

Fresh liver tissues were harvested and fixed in 4% paraformaldehyde for paraffin embedding. Embedded tissues were sectioned at 5 µm thickness and stained with hematoxylin and eosin (H&E). Results in FIG. 7 showed extensive diffuse hemorrhage and massive necrosis in the liver tissues of the control group, with severe disruption of hepatic lobular architecture. The miR-302b-3p agomir treatment group demonstrated significantly mitigated liver damage and more intact hepatic lobular structure, suggesting that miR-302b-3p had the therapeutic efficacy against liver injury.

### (3) Detection of Hepatocyte Apoptosis by Western Blotting

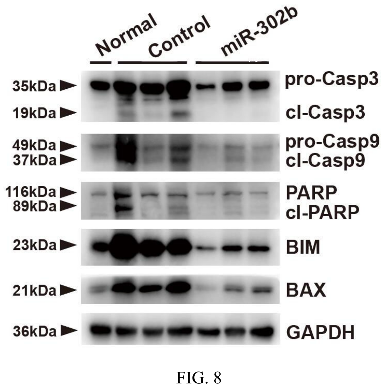

After liver collection, fresh liver tissues were used for protein extraction. Protein concentrations were quantified using a BCA protein assay kit, followed by Western blotting. Results in FIG. 8 indicated that miR-302b-3p reduced the cleavage and activation of apoptosis signaling pathways-related proteins Caspase-9/-3 and PARP and inhibited the expression of pro-apoptotic proteins BIM and BAX in liver tissues.

### VI. Summary

The miR-302 family is considered as a miRNA specifically expressed in embryonic stem cells, typically playing a crucial role in early development, with its expression rapidly declining after differentiation. Beyond this, the functions of the miR-302 family in other somatic cells have also been reported; however, it is hardly expressed in hepatocytes, and no studies on its role in hepatocytes or liver diseases have been documented. The present disclosure experimentally reveals that miR-302s exerts protective effects in acute liver failure mice by inhibiting hepatocyte apoptosis through targeting the pro-apoptotic gene Bcl2l11, thus providing a new idea for treating clinical liver failure or other liver-related diseases.

Technical features in the above embodiments may be combined in any combination. For simplicity of description, not all possible combinations of the technical features in the above embodiments are described. However, the combinations of the technical features are all to be considered as falling within the scope described in this specification provided that they do not conflict with each other.

The above examples only show several embodiments of the present disclosure, which are relatively specific and detailed, but should not be construed as limiting the scope of the present disclosure. It should be noted that various changes and modifications can be made by those of ordinary skill in the art without departing from the ideas of the present disclosure, and these changes and modifications are all within the scope of the present disclosure. Therefore, the scope of the present disclosure should be determined by the content of the appended claims.

## Claims

1. Use of a nucleic acid molecule in preparation of a medicament for treating a liver disease; the nucleic acid molecule satisfies one or more of the conditions as shown in items (a) to (e):
(a) the nucleic acid molecule comprises any one or more of miR-302 members;
(b) the nucleic acid molecule comprises a mimic or derivative of any one or more of miR-302 members, wherein the mimic or derivative has a core fragment as set forth in SEQ ID No. 1 and has the same or similar function as any one or more of miR-302 members;
(c) the nucleic acid molecule comprises a miRNA precursor, the miRNA precursor being processable into any one or more of miR-302 members, the mimic or derivative thereof in a host;
(d) the nucleic acid molecule comprises a polynucleotide fragment, the polynucleotide fragment being transcribable in the host to form the miRNA precursor; and
(e) the nucleic acid molecule comprises an expression vector containing the polynucleotide fragment.

2. Use according to claim 1, wherein the nucleic acid molecule comprises one or more selected from the group consisting of miR-302a-3p, miR-302b-3p, miR-302c-3p and miR-302d-3p.

3. Use according to claim 1 or 2, wherein the liver disease is acute liver failure.

4. Use according to any one of claims 1 to 3, wherein the medicament comprises the nucleic acid molecule and a pharmaceutically acceptable excipient.

5. Use according to any one of claims 1 to 4, wherein the medicament is in the form of a tablet, a capsule or granules.

6. Use according to any one of claims 1 to 4, wherein the medicament is in the form of a suspension, an oral solution, or an injection.

7. An in vitro method for protecting hepatocytes, comprising culturing hepatocytes using a culture medium supplemented with the nucleic acid molecule defined in any one of claims 1 to 6.

8. The in vitro method according to claim 7, wherein the hepatocytes are hepatocytes damaged by D-galactosamine induction.

9. The in vitro method according to claim 7 or 8, wherein the hepatocytes are mouse primary hepatocytes.

10. The in vitro method according to any one of claims 7 to 9, wherein the nucleic acid molecule has a concentration of about 10 nM to 100 nM.
